# EUROPÄISCHE PATENTSCHRIFT

(11) **EP 0 722 934 B1**
(45) Veröffentlichungstag und Bekanntmachung des Hinweises auf die Patenterteilung: **02.11.2000**
(21) Anmeldenummer: 96100158.3
(22) Anmeldetag: 08.01.1996
(51) Int. Cl.: C07D 213/61

(54) **Verfahren zur Herstellung von in 5-Stellung substituierten 2-Chlorpyridinen**
Process for the preparation of 5-substituted-2-chloropyridines
Procédé pour la préparation de pyridines-2-chloro-5-substitués

(30) Priorität: 19.01.1995 DE 19501478
(43) Veröffentlichungstag der Anmeldung: 24.07.1996
(73) Patentinhaber: BAYER AG, 51368 Leverkusen (DE)
(72) Erfinder: Jelig, Klaus, Dr., Overland Park, KS 66223 (US); Lindel, Hans, Dr., D-51373 Leverkusen (DE); Mannheims, Christoph, D-51373 Leverkusen (DE); Lantzsch, Reinhard, Dr., D-42115 Wuppertal (DE); Merz, Walter, Dr., D-51375 Leverkusen (DE)

(56) Entgegenhaltungen:
- EP-A- 0 490 199
- EP-A- 0 546 418
- DATABASE WPI Section Ch, Derwent Publications Ltd., London, GB; Class B, AN 66-31844F XP002003073 & SU-A-193 519 (POZNANSKAYA NL)

## Beschreibung

Die vorliegende Erfindung betrifft ein Verfahren zur Herstellung von in 5-Stellung substituierten 2-Chlorpyridinen durch Umsetzung von Dialkylformamiden mit Acetenamiden in Gegenwart von Chlorierungsmitteln.

Aus EP-OS 546 418 ist bekannt, daß 5-substituierte 2-Chlorpyridine aus Acetanamiden durch Umsetzung mit Dimethylformamid in Gegenwart eines Chlorierungsmittels zugänglich sind.

Dabei wird aus dem intermediär gebildeten Vilsmeiersalz das entsprechende Dimethylamin abgespalten, das im Prinzip wieder zum Formamid und in den Prozeß zurückgeführt werden könnte.

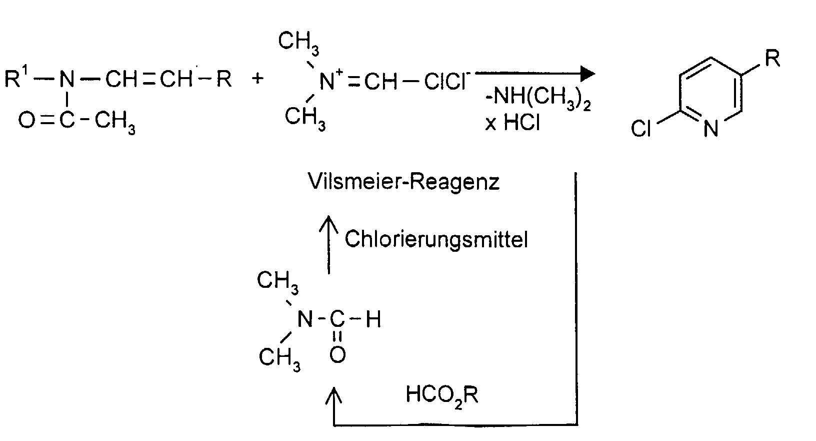

Das Dimethylamin entweicht zum Teil über die Gasphase. Zum Teil liegt es als Hydrochlorid vor und kann durch Reaktion mit dem Chlorierungsmittel verlorengehen:

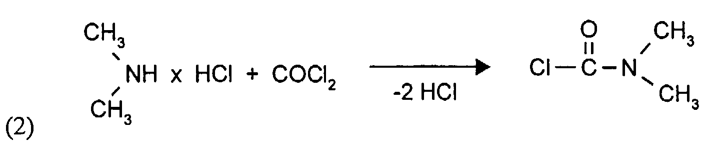

Dadurch wird die Ausbeute an rückführbarem Dimethylamin reduziert; außerdem sind die entstehenden Carbamidsäurechloride unerwünschte reaktive Nebenprodukte.

Gegenstand der vorliegenden Erfindung ist ein Verfahren zur Herstellung von in 5-Stellung substituierten 2-Chlorpyridinen der Formel (I) 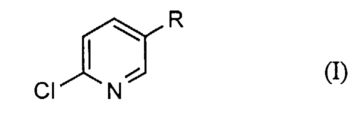 in welcher
- R: für gegebenenfalls substituiertes Alkyl oder Aralkyl steht,
durch Umsetzung von Acetenamiden der Formel (II) 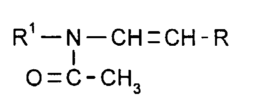 in welcher
- R: die oben angegebene Bedeutung hat
- R¹: für C₁-C₄-Alkyl oder Aryl-C₁-C₄-alkyl steht,
mit Vilsmeier-Reagenz, das durch Umsetzung von Formamiden der Formel (III) 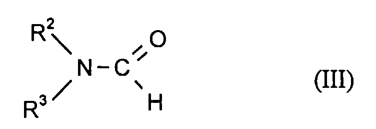 in welcher
- R² und R³: für geradkettiges, verzweigtes oder cyclisches C₄-C₈-Alkyl stehen,
mit einem Chlorierungsmittel, dessen Überschuß aus dem Reaktionsgemisch nach Beendigung der Reaktion des Vilsmeier-Reagenzes mit dem Acetenamid der Formel (II) destillativ oder durch Zusatz von Dialkylformamid der Formel (III) entfernt wird, erhalten wird und abschließendes Erhitzen des Reaktionsgemisches auf 80°C bis 160°C hergestellt wird, dadurch gekennzeichnet, daß Dialkylformamid, Acetenamid sowie ein Chorierungsmittel parallel in ein vorgelegtes Lösungsmittel dosiert werden.

Durch den erfindungsgemäßen Einsatz der Dialkylformamide der Formel (III) wird verhindert, daß die entsprechenden Dialkylamine über die Gasphase entweichen. Da diese Dialkylamine nicht mit Wasser mischbar sind lassen sie sich auch einfach vom Reaktionsgemisch abtrennen. Ihre Wiedergewinnung läßt sich noch dadurch steigern, daß man das Chlorierungsmittel, das nicht zur Bildung des Vilsmeier-Reagenzes reagiert hat, nach Beendigung der bestimmungsgemäßen Reaktion aus dem Reaktionsgemisch entfernt.

Das erfindungsgemäße Verfahren betrifft vorzugsweise die Herstellung von Verbindungen der Formel (I), in welcher
- R: für jeweils gegebenenfalls durch Fluor und/oder Chlor substituiertes C₁-C₆-Alkyl oder Phenyl-C₁-C₂-alkyl steht.

Insbesondere werden nach dem erfindungsgemäßen Verfahren Verbindungen der Formel (I) hergestellt, in welcher
- R: für Methyl, Ethyl oder Benzyl steht.

Die als Ausgangsstoffe zu verwendenden Acetenamide sind durch die Formel (II) allgemein definiert. In Formel (II) steht vorzugsweise
- R: für jeweils gegebenenfalls durch Fluor und/oder Chlor substituiertes C₁-C₆-Alkyl oder Phenyl-C₁-C₂-alkyl, und
- R¹: für jeweils gegebenenfalls durch Fluor und/oder Chlor substituiertes C₁-C₄-Alkyl oder Benzyl.
Insbesondere stehen in Formel (II)
- R: für Methyl, Ethyl oder Benzyl und
- R¹: für Benzyl.

Die Ausgangsstoffe der Formel (II) sind bekannt und/oder können nach an sich bekannten Verfahren hergestellt werden (vgl. J. Chem. Soc. Perkin Trans. I 1984, 1173-1182).

Man erhält die Acetenamide der Formel (II) beispielsweise, wenn man Imine der allgemeinen Formel (IV)

R¹-N=CH-CH₂-R (IV)

in welcher
- R und R¹: die oben angegebene Bedeutung haben
mit Acetanhydrid oder Acetylchlorid, gegebenenfalls in Gegenwart eines Säureakzeptors, wie z.B. Triethylamin, und gegebenenfalls in Gegenwart eines Verdünnungsmittels, wie z.B. Toluol, bei Temperaturen zwischen 0°C und 50°C umsetzt und nach üblichen Methoden aufarbeitet.

Die Imine der Formel (IV) sind bekannt und/oder können nach an sich bekannten Verfahren hergestellt werden (vgl. J. Am. Chem. Soc. 66 (1944), 82-84).

Chlorierungsmittel, die beim erfindungsgemäßen Verfahren eingesetzt werden können, sind Verbindungen, welche mit Formamiden das sogenannte Vilsmeier-Reagenz (N,N-disubstituiertes Chlormethylimmoniumchlorid) (V) 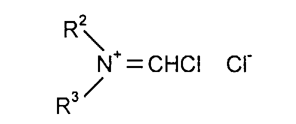 wobei
- R² und R³: die oben angegebene Bedeutung haben
bilden.

Als Formamide kommen in Frage Di-n-butylformamid, Di-iso-, Di-sek-butylformamid, Dicyclohexylformamid. Bevorzugt ist N,N-Di-n-butylformamid.

Zu den Chlorierungsmitteln gehören insbesondere Säurechloride die destillativ aus dem Reaktionsgemisch entfernt werden können, wie Phosphorylchlorid (Phosphoroxychlorid/POCl₃), Phosgen, Oxalylchlorid und Thionylchlorid. Besonders bevorzugt wird Phosgen.

Das erfindungsgemäße Verfahren zur Herstellung der 5-substituierten 2-Chlor-pyridine der Formel (I) wird in Gegenwart eines Verdünnungsmittels durchgeführt. Es kommen praktisch alle für die Reaktion inerten organischen Lösungsmittel in Betracht. Hierzu gehören vorzugsweise aliphatische und aromatische, gegebenenfalls halogenierte Kohlenwasserstoffe wie Hexan, Heptan, Cyclohexan, Methylcyclohexan, Benzol, Toluol, Xylol, Chlorbenzol, o-Dichlorbenzol, Chloroform und Tetrachlormethan, sowie Ether wie Methyl-tert.-butylether, Methyl-tert.-Amylether und 1,2-Dimethoxyethan, sowie Nitrile wie Acetonitril, Propionitril, n- oder iso-Butyronitril. Besonders bevorzugt ist Toluol und Chlorbenzol.

Die Reaktionstemperaturen können bei dem erfindungsgemäßen Verfahren in einem größeren Bereich variiert werden. Man arbeitet bei Temperaturen zwischen -30°C und +160°C, vorzugsweise bei Temperaturen zwischen -10°C und +145°C, insbesondere in der ersten Umsetzungsphase bei 0°C bis 40°C, dann bei 80°C bis 145°C.

Das erfindungsgemäße Verfahren wird im allgemeinen unter Normaldruck durchgeführt. Es ist jedoch auch möglich, unter erhöhtem oder vermindertem Druck zwischen 0,1 bar und 10 bar zu arbeiten.

Zur Durchführung des erfindungsgemäßen Verfahrens setzt man auf 1 Mol Acetenamid der Formel (II) im allgemeinen zwischen 1 und 10 Mol, vorzugsweise zwischen 1,5 und 5 Mol, insbesondere zwischen 2,0 und 3,0 Mol Chlorierungsmittel und zwischen 1 und 50 Mol, vorzugsweise zwischen 1 und 1,5 Mol Dialkylformamid ein.

Die Reagentien werden parallel dosiert, z.B. parallele Dosierung des Gemisches aus Dialkylformamid und dem Acetenamid und Phosgen zu vorgelegtem Lösungsmittel. Dadurch ist es möglich hoch-konzentriert zu arbeiten - auch bei Verwendung von Lösungsmitteln wie Chlorbenzol oder Toluol - da Feststoff-Ausfällungen vermieden werden. Das Chlorierungsmittel reagiert unter diesen Bedingungen praktisch dosierkontrolliert ab, wodurch eine Akkumulation mit entsprechendem Gefahrenpotential vermieden wird. Da das Auftreten kristalliner Phasen vermieden wird, ist es ebenfalls möglich, eine kontinuierliche Reaktionsführung, z.B. in einer Reaktorkaskade, anzuwenden.

Die parallele Dosierung ist in verschiedener Weise möglich. Das Acetenamid kann im Gemisch mit dem Dialkylformamid parallel zur Einleitung des Chlorierungsmittels zu vorgelegtem Lösungsmittel dosiert werden.

Ebenso ist es möglich, die Reagenzien getrennt aber parallel zu dosieren.

Es kann aber auch Dialkylformamid ins Lösungsmittel vorgelegt und Chlorierungsmittel sowie das Acetenamid parallel zudosiert werden.

Die Dosierungen werden in einem Temperaturbereich von etwa -10°C bis +50°C durchgeführt, besonders bevorzugt: +10°C bis +40°C.

Anschließend wird noch für eine Zeit von 0,5 bis 5 Stunden, besonders bevorzugt: 0,5 bis 2 Stunden bei einer Temperatur von unter 50°C gerührt.

Nach Dosierung der Reagentien wird bevorzugt bei Temperaturen unter 50°C nachgerührt, bis die bestimmungsgemäße Reaktion des Chlorierungsmittels abgeschlossen ist. Danach wird das Chlorierungsmittel destillativ entfernt. Dazu wird das Reaktionsgemisch zum Sieden gebracht und das überschüssige Chlorierungsmittel - rein oder als Gemisch mit dem verwendeten Lösungsmittel - abdestilliert. Dies wird bewerkstelligt durch Anlegen eines ausreichenden Vakuums am Reaktor bei relativ tiefer Temperatur (unter 50°C) oder indem das Gemisch über eine Destillationskolonne im Siedezustand bei kurzer Verweilzeit in der Kolonne in einen weiteren Reaktor dosiert wird, wobei das Chlorierungsmittel über die Kolonne abdestilliert wird. Der zur Destillation erforderliche Siedezustand wird durch Anlegen eines geeigneten Vakuums in der Kolonne bei entsprechendem Temperaturprofil oder bei Normaldruck durchgeführt.

Eine weitere, weniger bevorzugte Entfernung überschüssigen Chlorierungsmittels besteht in der Zugabe eines Dialkylformamids zur chemischen Abreaktion. Bevorzugt ist dabei das Nachsetzen einer bestimmten Menge des ohnehin zur Reaktion eingesetzten Dialkylformamids, wodurch freies Chlorierungsmittel zum Vilsmeier-Reagenz abreagiert und dadurch der Reaktion mit dem Dialkylamin entzogen wird. Aus dem so gebildeten, überschüssigen Vilsmeier-Komplex, kann das Dialkylformamid nach der wäßrigen Aufarbeitung isoliert und zurückgewonnen werden. Diese Variante ist auch für nicht destillierbare Chlorierungsmittel geeignet.

Im Anschluß an die Entfernung des überschüssigen Chlorierungsmittels wird die Reaktion bei entsprechender Temperatur (80 bis 160°C, besonders bevorzug 100 bis 145°C) abgeschlossen.

### Beispiel 1

Zu 700 ml und Chlorbenzol werden bei 40°C Innentemperatur parallel
a) ein Gemisch aus 189 g (1 mol) N-Benzyl-N-(l-propenyl)-acetamid und 172,6 g (1,1 mol) N,N-Di-n-butylformamid und
b) 297 g (3 mol) Phosgen dosiert.

Das Gemisch wird für 1 Stunde bei 40°C gerührt. Anschließend wird das Gemisch gleichmäßig über eine Destillationskolonne in einen Reaktionskolben mit 100 ml siedendem Chlorbenzol dosiert. Die Dosierung erfolgt in der Mitte der Kolonne, am Kopf wird kontinuierlich ein Gemisch aus Chlorbenzol und Phosgen abdestilliert.
- Bedingungen:: R:F = 10:1
550 mbar

Nach beendeter Dosierung wird für 1 Stunde bei 115°C gerührt und das Rohgemisch (811 g) gegen Standard analysiert:
2-Chlor-5-methylpyridin: 14,3 % (91 % d.Th.)
Di-n-butylcarbamidsäurechlorid: 0,4 % (1,7 % d.Th.)
Di-n-butylamin x HCl: 18,8 % (91 % d.Th.).

## Patentansprüche

1. Verfahren zur Herstellung von in 5-Stellung substituierten 2-Chlorpyridinen der Formel (I) 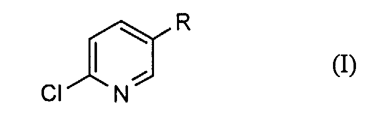 in welcher
R für gegebenenfalls substituiertes Alkyl oder Aralkyl steht,
durch Umsetzung von Acetenamiden der Formel (II) 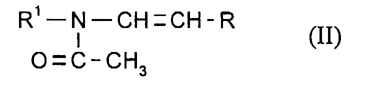 in welcher
R die oben angegebene Bedeutung hat
R¹ für C₁-C₄-Alkyl oder Aryl-C₁-C₄-alkyl steht,
mit Vilsmeier-Reagenz, das durch Umsetzung von Formamiden der Formel (III) 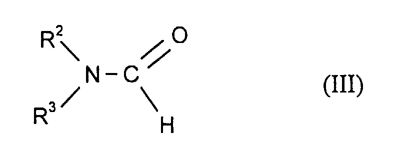 in welcher
R² und R³ für geradkettiges, verzweigtes oder cyclisches C₄-C₈-Alkyl stehen
mit einem Chlorierungsmittel, dessen Überschuß aus dem Reaktionsgemisch nach Beendigung der Reaktion des Vilsmeier-Reagenzes mit dem Acetenamid der Formel (II) destillativ oder durch Zusatz von Dialkylformamid der Formel (III) entfernt wird, erhalten wird und abschließendes Erhitzen des Reaktionsgemisches auf 80°C bis 160°C hergestellt wird, dadurch gekennzeichnet, daß Dialkylformamid, Acetenamid sowie ein Chlorierungsmittel parallel in ein vorgelegtes Lösungsmittel dosiert werden.

2. Verfahren nach Anspruch 1, dadurch gekennzeichnet, daß das Molverhältnis von Acetenamid der Formel (II) und Dialkylformamid der Formel (III) zwischen 1 zu 1 und 1 zu 1,5 liegt.

3. Verfahren nach Anspruch 1, dadurch gekennzeichnet, daß ein Gemisch aus Dialkylformamid und Acetenamid sowie Phosgen parallel in ein vorgelegtes Lösungsmittel dosiert werden.

4. Verfahren nach Anspruch 1, dadurch gekennzeichnet, daß nach beendeter Dosierung überschüssiges Chlorierungsmittel unterhalb von 50°C destilliert wird.

5. Verfahren nach Anspruch 1, dadurch gekennzeichnet, daß das Verfahren kontinuierlich durchgeführt wird.

## Claims

1. Process for the preparation of 5-substituted 2-chloropyridines of the formula (I) 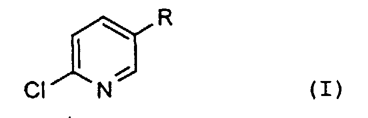 in which
R represents optionally substituted alkyl or aralkyl,
by reaction of acetenamides of the formula (II) 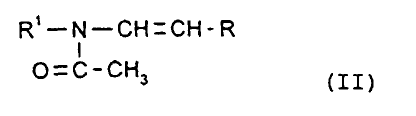 in which
R has the meaning indicated above
R¹ represents C₁-C₄-alkyl or aryl-C₁-C₄-alkyl,
with Vilsmeier reagent, which is obtained by reaction of formamides of the formula (III) 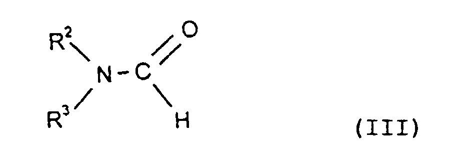 in which
R² and R³ represent straight-chain, branched or cyclic C₄-C₈-alkyl
with a chlorinating agent, the excess of which is removed from the reaction mixture by distillation or by addition of dialkylformamide of the formula (III) after completion of the reaction of the Vilsmeier reagent with the acetenamide of the formula (II), and is prepared subsequent heating of the reaction mixture to from 80°C to 160°C, characterized in that dialkylformamide, acetenamide and a chlorinating agent are added in parallel to an initially introduced solvent.

2. Process according to Claim 1, characterized in that the molar ratio of acetenamide of the formula (II) and dialkylformamide of the formula (III) is between 1 to 1 and 1 to 1.5.

3. Process according to Claim 1, characterized in that a mixture of dialkylformamide and acetenamide and also phosgene are added in parallel to an initially introduced solvent.

4. Process according to Claim 1, characterized in that, after addition is complete, excess chlorinating agent is distilled below 50°C.

5. Process according to Claim 1, characterized in that the process is carried out continuously.

## Revendications

1. Procédé de production de 2-chloropyridines substituées en position 5, de formule (I) 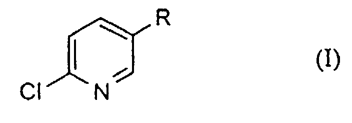 dans laquelle
R est un groupe alkyle ou aralkyle éventuellement substitué,
par réaction d'acéténamides de formule (II) 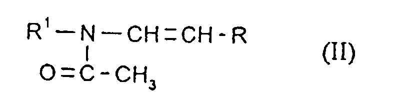 dans laquelle
R a la définition indiquée ci-dessus
R¹ est un groupe alkyle en C₁ à C₄ ou aryl-(alkyle en C₁ à C₄),
avec le réactif de Vilsmeier, que l'on obtient par réaction de formamides de formule (III) 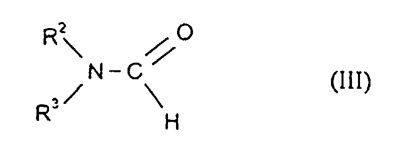 dans laquelle
R² et R³ représentent un groupe alkyle en C₄ à C₈ linéaire, ramifié ou cyclique
avec un agent de chloration dont l'excès est éliminé du mélange réactionnel par distillation ou par addition de dialkylformamide de formule (III) après la fin de la réaction du réactif de Vilsmeier avec l'acéténamide de formule (II), puis chauffage du mélange réactionnel à une température de 80°C à 160°C, caractérisé en ce que le dialkylformamide, l'acéténamide de même qu'un agent de chloration sont dosés parallèlement dans un solvant introduit préalablement.

2. Procédé suivant la revendication 1, caractérisé en ce que le rapport molaire de l'acéténamide de formule (II) au dialkylformamide de formule (III) se situe entre 1:1 et 1:1,5.

3. Procédé suivant la revendication 1, caractérisé en ce qu'un mélange de dialkylformamide et d'acéténamide ainsi que de phosgène est dosé parallèlement dans un solvant introduit préalablement.

4. Procédé suivant la revendication 1, caractérisé en ce que, lorsque le dosage est terminé, l'agent de chloration en excès est distillé en dessous de 50°C.

5. Procédé suivant la revendication 1, caractérisé en ce qu'il est mis en oeuvre en continu.
